(19) Europäisches Patentamt
European Patent Office
Office européen des brevets

(11) Publication number: **0 137 415**
**B1**

(12) # EUROPEAN PATENT SPECIFICATION

(45) Date of publication of patent specification: **01.06.88**

(21) Application number: **84111502.5**

(22) Date of filing: **26.09.84**

(51) Int. Cl.[4]: **C 07 C 121/34,**
C 07 C 121/40,
C 07 D 213/64, C 07 D 213/61

(54) **Addition of polyhalonitriles to functionalized olefins.**

(30) Priority: **26.09.83 US 536063**

(43) Date of publication of application:
**17.04.85 Bulletin 85/16**

(45) Publication of the grant of the patent:
**01.06.88 Bulletin 88/22**

(84) Designated Contracting States:
**BE CH DE FR GB IT LI NL SE**

(56) References cited:
**EP-A-0 046 735**
**EP-A-0 119 683**
**DE-B-1 154 453**
**FR-A-1 409 516**
**US-A-4 468 354**

**JOURNAL OF ORGANIC CHEMISTRY, vol. 31, no. 1, January 1966, pages 3000-3003; S. MURAI et al.: "Copper salts catalyzed addition of trichloro- and dichloroacetonitriles to olefins"**

(73) Proprietor: **THE DOW CHEMICAL COMPANY**
**2030 Dow Center Abbott Road P.O. Box 1967**
**Midland, MI 48640 (US)**

(72) Inventor: **Lysenko, Zenon**
**214 West Meadowbrook Drive**
**Midland Michigan 48640 (US)**
Inventor: **Pews, Richard Garth**
**4402 Andre Street**
**Midland Michigan 48640 (US)**

(74) Representative: **Weickmann, Heinrich, Dipl.-Ing. et al**
**Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820**
**D-8000 München 86 (DE)**

**EP 0 137 415 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

European Patent 46735 (Ciba-Geigy) discloses the preparation of chloropyridines substituted by methyl, trichloromethyl or trifluoromethyl groups by the addition of trichloroacetaldehyde to methacrylonitrile or α-trifluoromethacrylonitrile, the addition of 2,2-dichloropropionaldehyde, pentachloropropionaldehyde, 2,2-dichloro-3,3,3-trifluoropropionaldehyde to acrylonitrile or the addition of 2,2-dichloropropionaldehyde to methacrylonitrile and cyclizing the resulting intermediate.

DE—B—1 154 453 describes a process for the preparation of functional derivatives of α,α,α'-trihaloglutaric acid by addition of derivatives of dihaloacetic acid to α,β-unsaturated carboxylic acids or derivatives thereof, which are substituted with a halogen in the α-position.

Further Fr—A—1 409 516 describes the addition of halogenic compounds to the double bonds of olefins using heavy metal compounds as catalysts.

The use of copper salts as catalysts for the addition of trichloro- and dichloro acetonitriles to olefins is described in J. Org. Chem. *31*, (1966), 3000—3003.

EP—A—0 119 683 (act 54(3)), as well as the corresponding application US—A—4 468 354, describes a continuous process for the preparation of 5-oxo-2,4-dichloro-4-substituted pentane nitriles by reacting an α,α-dichloroaldehyd with an acrylonitrile in the presence of a transition metal catalyst in a coil reactor.

We have now found that compounds having the formula

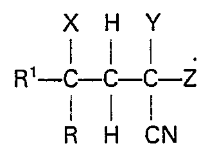

wherein R is CHO or $COR^2$ where $R^2$ is halo, alkyl having 1 to 6 carbon atoms or alkoxy having 1 to 6 carbon atoms;

$R^1$ is H or lower alkyl having 1 to 6 carbon atoms;

X is Br, Cl or I;

Y is Cl, Br, $CF_3$, alkyl of 1 to 6 carbon atoms, substituted or unsubstituted aryl, preferably having 6 to 10 carbon atoms or a heterocyclic radical having 5 or 6 atoms in the ring; and

Z is H, F or Cl, are readily prepared by a process which comprises reacting an acetonitrile having the formula

$$C(X^1)_3CN$$

where $X^1$ is any combination of H, F, Cl, Br or I, with the proviso that there be no more than one H, with an olefinic compound having the formula

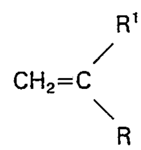

wherein R and $R^1$ are as above-identified in the presence of a catalyst, which comprises a complex of cuprous chloride, ruthenium chloride or rhodium chloride and triphenylphosphine or a complex of cuprous chloride and tri-n-butylphosphine.

The process of this invention utilizes polyhalogenated acetonitriles which have demonstrated increased reactivity toward acrylates and acroleins, thus enabling the use of milder reaction conditions than processes of the known art.

The reaction may be carried out neat or, if desired, an inert solvent, e.g., an alkyl nitrile, may be employed. The reaction is generally carried out using equimolar amounts of the trihaloacetonitrile and olefin, e.g., acryloyl chloride, methacrolein or acrolein, although a slight excess of the lower boiling reactant may be employed as a solvent if desired.

The reaction may be carried out at a temperature of from 40° to 200°C, but is preferably carried out at a temperature of from 70° to 90°C.

This invention is further illustrated by the following examples.

Preparation of dibromoacetonitrile

Cyanoacetic acid (85 g, 1.0 mole) was dissolved in 1.5 liter of $H_2O$ and cooled to 10°C. N-bromosuccinimide (356 g, 2.0 moles) was added in small portions to the stirring solution. After a brief induction period, a vigorous evolution of $CO_2$ gas had begun. Upon completion of the addition, the reaction mixture was allowed to stir at room temperature for a period of one hour after which the reaction mixture was poured into a 2-liter separatory funnel to allow the separation of dibromoacetonitrile. The aqueous portion was then extracted with 3×150 ml portions of methylene chloride which were combined with the

dibromoacetonitrile already separated. The combined extracts were dried over $MgSO_4$ and the solvent was removed *in vacuo*. The residue was distilled to afford 167 g (84 percent yield) of dibromoacetonitrile, b.p. 60°C at 20 mmHg, as a pale yellow liquid.

Preparation of dichloroacetonitrile

Cyanoacetic acid (170 g, 2.0 moles) was dissolved in 2 liters of water and cooled to 10°C. N-chlorosuccinimide (53 g, 4.0 moles) was added slowly to the stirred solution. After a brief induction period, a vigorous evolution of $CO_2$ began. Addition of N-chlorosuccinimide was maintained such that the temperature did not exceed 35°C. Upon completion of the addition, the reaction mixture was stirred at room temperature for a period of one hour and allowed to stand. The separated organic phase was removed and the aqueous phase was extracted with 3×200 ml portions of $CH_2Cl_2$. The extracts were combined with the product and dried over $MgSO_4$. The solvent was removed *in vacuo* and the remaining liquid was distilled to afford 160.6 g (75 percent yield) of dichloroacetonitrile, b.p. 112°—113°C.

Example 1

Preparation of 4-formyl-2,2,4-trichlorovaleronitrile

A solution of 150 ml of $CCl_3CN$ (1.5 moles) and 80 ml of methacrolein (1.0 mole) containing 10 g of CuCl and 0.5 g triphenylphosphine catalyst was heated to reflux in a 500 ml round bottomed flask equipped with a spiral condenser for a period of 4.5 hours. During that time, the reaction temperature rose from 75°C to 85°C. At the end of this time the unreacted materials were removed *in vacuo* to afford 174 g of a dark red oil (84 percent). Distillation of this residue afforded 150 g, b.p. 75°C (1.5—3.0 mm Hg) of a yellow liquid (83 percent) with greater than 95 percent purity by G. C. HNMR and CNMR. 'Hnmr (Acetone $d_6$, TMS); δ 1.90 (S 3H —$CH_3$); δ 3.60 (S 2H —$CH_2$—); δ 9.58 (S 1H —CHO). IR NaCl neat 1730 $cm^{-1}$ 2255 $cm^{-1}$.

| Elemental analysis | C | H | N |
|---|---|---|---|
| Calculated for $C_6H_6Cl_3NO$: | 33.60 | 2.82 | 6.53 |
| Found: | 33.50 | 2.88 | 6.44 |

Example 2

Preparation of 2,4,4-trichloro-4-cyanopentanoyl chloride

To a 100 ml round bottomed flask was added 10 ml (14.0 g) of $CCl_3CN$, 16 ml $H_2C{=}CHCOCl$ (22 g, 0.20 mole), 0.5 g CuCl and 0.025 g triphenylphosphine catalyst. The flask was equipped with a spiral reflux condenser topped with a $CaCl_2$ drying tube and the reaction mixture was heated at reflux for a period of 2½ hours. At this time, G.C. analysis showed that the reaction was at 50 percent converted (by area percent), heat was removed and unreacted starting materials were stripped off *in vacuo* to afford a dark red viscous oil. This resulting residue was transferred to a Kugelrohr apparatus and distilled at 90°C/4 mm Hg to afford 5 g of product in 21.3 percent overall yield.

Example 3

Preparation of 2,2,4-trichloro-5-oxypentanenitrile

(A) Following the above general procedures, a mixture of 56 g (1.0 mole) of acrolein and 72 g (0.5 mole) of trichloroacetonitrile was heated to reflux (60°C) in the presence of 100 mg of CuCl and 100 mg of triphenylphosphine. The reaction was kept at reflux for 15 hours under nitrogen by which time the temperature had risen to 85°C. After cooling to ambient temperature, unreacted starting materials were removed in vacuo and the residue distilled from a Kugelrohr apparatus to provide the titled product (50 g, 49.8 percent yield), purity 84 percent by gas chromatography.

(B) A solution of 56 g (1.0 mole) of acrolein and 214 g (1.5 moles) of trichloroacetonitrile was heated to reflux (60°C) in the presence of 100 mg of CuCl and 100 mg of triphenylphosphine. The reaction was kept at reflux for 1.5 hours under dry nitrogen by which time the temperature had risen to 85°C. After cooling to room temperature, unreacted starting materials were removed *in vacuo* and the residue distilled through a Kugelrohr apparatus to provide 2,2,4-trichloro-5-oxopentanenitrile (170 g, 85 percent yield) as an amber liquid, b.p. 65°C, 0.95 mmHg. 'Hnmr (Acetone $d_6$, TMS) δ 3.39 (dq 2H —$CH_2$—); δ 4.94 (dd 1H —C*H*Cl); δ 9.58 (S 1H CHO). IR NaCl neat 1735 $cm^{-1}$ 2250 $cm^{-1}$.

| Elemental analysis: | C | H | N |
|---|---|---|---|
| Calculated for $C_5H_4Cl_3NO$: | 29.96 | 2.01 | 6.99 |
| Found: | 30.10 | 2.11 | 6.99 |

Example 4

Preparation of 2,4-dichloro-4-formalyvaleronitrile

A solution of 11 grams (0.1 mole) of dichloroacetonitrile and 14.0 grams (0.2 mole) of methacrolein in 50 ml of propionitrile containing 100 mg of CuCl and 200 mg of triphenylphosphine was heated to reflux (85°—94°C) for 18 hours under a nitrogen blanket. After cooling to ambient temperature the solvent and

unreacted starting materials were removed in vacuo and the residue was distilled in a Kugelrohr apparatus at 100°C and 5 mm Hg to afford 10 grams of the titled product. NMR indicated a mixture of diastereoisomers with traces of 2,3-dichloro-5-methylpyridine. 'Hnmr ($CDCl_3$, TMS) δ 1.78 (S 3H $CH_3$); δ 2.3—3.10 (m 2H —$CH_2$—); δ 4.75 (t 1H —C*H*Cl—); δ 95 (S 1H —C*H*O). IR NaCl neat 2250 $cm^{-1}$ 1750 $cm^{-1}$; b.p. 90°C at 5.0 mmHg.

Example 5
Preparation of 2,4-dichloro-4-methyl-5-oxopentane nitrile and 2,3-dichloro-5-methylpyridine

A solution of dichloroacetonitrile (11.0 g, ~0.1 mole) and methacrolein (14.0 g, 0.20 mole) in 50 ml of propionitrile containing 200 mg of CuCl and 200 mg of tri-n-butylphosphine was placed in a Carius tube and heated to 130°C for a period of six hours. Upon completion, the reaction was cooled to room temperature and the solvent and unreacted starting materials were removed *in vacuo*. The residue was distilled through a Kugelrohr apparatus at 100°—120°C at 5 mmHg to afford a pale yellow liquid which slowly crystallized upon standing. The white crystals were washed with cold hexane and dried to afford 6.5 g, (40 percent yield) of 2,3-dichloro-5-methylpyridine, m.p. 42°—45°C. Evaporation of the hexane washes afforded 2,4-dichloro-4-methyl-5-oxopentanenitrile, 1.6 g, 10% additional yield for a total yield of 50%. 'Hnmr ($CCl_4$, TMS) δ 2.35 (S 3H —$CH_3$), δ 7.62 (d 1H pyr C-4H); δ 8.16 (d 1H pyr C-6H).

Example 6
Preparation of 2,3-dibromo-5-methylpyridine by way of the *in situ* formation and ring closure of 2,4-dibromo-4-formyl valeronitrile

A solution of 19.0 g (~0.1 mole) of dibromoacetonitrile and 14 g (0.2 mole) of methacrolein in 50 ml of propionitrile containing 100 mg of CuCl and 200 mg of tri-n-butylphosphine was heated to reflux (90°C) under $N_2$ atmosphere for 14.5 hours. After this time, the solvent and unreacted starting materials were removed *in vacuo*. 'Hnmr of the reaction mixture revealed the presence of 2,4-dibromo-4-formylvaleronitrile present as a mixture of diastereomers in an appropriate 1:1 ratio (by gas chromatography). 'Hnmr ($CDCl_3$, TMS), δ 2.55 (S 3H —*$CH_3$*); δ 2.7—3.3 (M 2H —$CH_2$); δ 4.80 (t 1H —CHBr—); δ 9.5 (S 1H —CHO).

Attempts to isolate the 2,4-dibromo-4-formyl-valeronitrile by vacuum distillation resulted in the conversion of this compound to 2,3-dibromo-5-methyl-pyridine. Thus, the residue was placed on a Kugelrohr apparatus and distilled at 100°C and 5 mm of Hg to afford 11.5 g (49% isolated yield) of a pale yellow oil which slowly solidified upon standing. Recrystallization from hexane afforded 2,3-dibromo-5-methylpyridine as colorless platelets, m.p. 53°—55°C.

| Elemental analysis: | C | H | N |
|---|---|---|---|
| Calculated for $C_6H_5Br_2N$: | 28.72 | 2.01 | 5.58 |
| Found: | 29.00 | 2.00 | 5.58 |

Example 7
Preparation of 2,2,4-trichloro-5-oxo-hexanenitrile

A solution of 28 grams (0.2 mole) trichloroacetonitrile and 20 grams (0.3 mole) methylvinylketone in 50 ml of propionitrile containing 1.0 gram of CuCl and triphenylphosphine (1:1 ratio by weight) was refluxed overnight. Solvent was removed in vacuo and the residue distilled in a Kugelrohr apparatus to afford 30 grams of titled product having a boiling point of 100°C at 3 mm Hg. 'Hnmr (Acetone $d_6$ TMS); δ 2.42 (S 3H $CH_3$); δ 3.34 (dq 2H —$CH_2$—); δ 4.90 (dd 1H —CHCl—). b.p. 72°—73°C; 0.8 mmHg. IR NaCl neat 1725 $cm^{-1}$ 2255 $cm^{-1}$.

| Elemental analysis: | C | H | N |
|---|---|---|---|
| Calculated for $C_6H_6Cl_3NO$: | 33.60 | 2.82 | 6.53 |
| Found: | 33.50 | 2.75 | 6.70 |

Example 8
Preparation of 2,4-dibromo-2-fluoro-4-formylvaleronitrile and its conversion to 2-bromo-3-fluoro-5-methylpyridine and 2-bromo-3-fluoro-5-methylpyridine hydrobromide

Dibromofluoroacetonitrile (5.0 g, 0.025 mole) and 3.5 g (0.05 mole) of methacrolein were dissolved in 50 ml of propionitrile containing 50 mg of CuCl, 50 µl of n-$Bu_3P$ and 50 µl of triethylamine. This solution was placed in a Carius tube and heated at 100°C for a period of 9 hours. Upon completion, the solvent and unreacted starting material were removed under reduced pressure. An 'Hnmr spectrum of the material showed the presence of diastereomeric 2,4-dibromo-2-fluoro-4-formylvaleronitrile along with trace amounts of 2-bromo-3-fluoro-5-methylpyridine. 'Hnmr (acetone $d_6$): δ 1.95, 2.0 (singlets, 3H, —$CH_3$); δ 3.2—3.65 (m, 24, —$CH_2$); δ 9.20, 9.35 (doublets, 1H, CHO). Distillation of this residue through a Kugelrohr apparatus at 120°C and 3 mmHg afforded only a mixture of pyridines. The distillate which contained a precipitate was taken up in hot hexane and filtered to remove the precipitate. This precipitate was dried and purified by sublimation (50°—80°C at 0.5 mm Hg) to afford 0.795 g (11.7 percent yield) of 2-bromo-3-fluoro-5-methylpyridine hydrobromide, m.p. 198°—203°C (sublimed). 'Hnmr (acetone $d_6$: $D_2O$; 1:1): δ 2.45 (s, 3H, —$CH_3$); δ 7.75 (double doublet, 1H, pyr-4H); JHF 9.4 Hz; δ 8.15 (broad singlet, 1H, pyr-6H). Removal of the hexane *in vacuo* afforded a colorless oil which solidified on standing. Sublimation of this material (30°C at 0.3 mm Hg) gave 0.95 g (20.0 percent yield) of 2-bromo-3-fluoro-5-methylpyridine, m.p. 32°—33°C. 'Hnmr (acetone $d_6$): δ 2.35 (s, 3H, —$CH_3$); δ 7.50 (double doublet, 1H, pyr-4H), JHF 9.3 Hz; δ 8.10 (broad s, 1H, pyr-6H).

Elemental analysis

| | |
|---|---|
| Calc'd for $C_6H_6Br_2FN$: | C, 26.59; H, 2.23; N, 5.17. |
| Found: | C, 26.30; H, 2.54; N, 5.29. |
| Calc'd for $C_6H_5BrFN$: | C, 37.92; H, 2.65; N, 7.37. |
| Found: | C, 37.24; H, 2.85; N, 6.98. |

Example 9

Preparation of 4-formyl-2,4-dichloro-2-fluorovaleronitrile

The above general procedures were repeated except to employ 12.8 grams (0.1 mole) of dichlorofluoroacetonitrile and 14.0 grams (0.2 mole) of methacrolein. The catalyst comprised 100 mg CuCl, 50 microliters of tri-n-butylphosphine and 50 microliters of triethylamine. After 9 1/2 hours at 110°C, the unreacted starting materials and solvent were removed in vacuo and the residue distilled from a Kugelrohr apparatus (95°C at 3.0 mm Hg) to provide 12 grams of the title product. NMR revealed the sample to be a mixture of diastereoisomers.

**Claims**

1. A process for making a compound having the formula

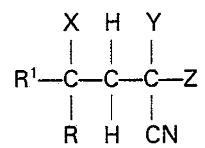

wherein

R is CHO or $COR^2$ where $R^2$ is halo, alkyl having 1 to 6 carbon atoms or alkoxy having 1 to 6 carbon atoms;

$R^1$ is H or lower alkyl having 1 to 6 carbon atoms;

X is Br, Cl, or I;

Y is Cl, Br, $CF_3$, alkyl of 1 to 6 carbon atoms, substituted or unsubstituted aryl, preferably having 6 to 10 carbon atoms or a heterocyclic radical having 5 to 6 atoms in the ring; and

Z is H, F or Cl,

which comprises reacting an acetonitrile having the formula

$$C(X^1)_3CN$$

where

$X^1$ is any combination of H, Cl, Br, I or F, with the proviso that there be no more than one H, with an olefinic compound having the formula

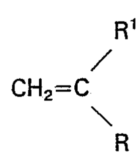

wherein

$R^1$ is H or lower alkyl having 1 to 6 carbon atoms; and

R is CHO or $COR^2$ where $R^2$ is halo, alkyl having 1 to 6 carbon atoms or alkoxy having 1 to 6 carbon atoms;

in the presence of a catalyst, which comprises a complex of cuprous chloride, ruthenium chloride or rhodium chloride and triphenylphosphine or a complex of cuprous chloride and tri-n-butylphosphine.

2. Process of claim 1 wherein the reaction is carried out at a temperature of from 40° to 200°C.

3. Process of claim 2 wherein the temperature is from 70° to 90°C.

4. Process of claim 1 to 3 wherein the reaction is carried out neat.

5. Process of claim 1 wherein the reactants are employed in approximately equal molar proportions.

**Patentansprüche**

1. Verfahren zur Herstellung einer Verbindung mit der Formel

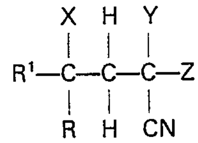

worin

R CHO oder $COR^2$ ist, wobei $R^2$ Halo, Alkyl mit 1 bis 6 Kohlenstoffatomen oder Alkoxy mit 1 bis 6 Kohlenstoffatomen ist;

$R^1$ H oder ein niedriges Alkyl mit 1 bis 6 Kohlenstoffatomen ist;

X Br, Cl, oder I ist;

Y Cl, Br, $CF_3$, Alkyl mit 1 bis 6 Kohlenstoffatomen, substituiertes oder unsubstituiertes Aryl, bevorzugt mit 6 bis 10 Kohlenstoffatomen oder ein heterocyclischer Rest mit 5 oder 6 Atomen im Ring ist; und

Z H, F oder Cl ist,

umfassend zur Reaktion bringen eines Acetonitrils mit der Formel

$$C(X^1)_3CN$$

worin

$X^1$ jegliche Kombination von H, Cl, Br, I oder F ist, mit der Maßgabe, daß nicht mehr als ein H vorhanden ist, mit einer olefinischen Verbindung mit der Formel

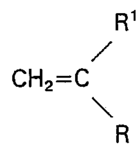

worin

$R^1$ H oder ein niedriges Alkyl mit 1 bis 6 Kohlenstoffatomen; und

R CHO oder $COR^2$ ist, wobei $R^2$ Halo, Alkyl mit 1 bis 6 Kohlenstoffatomen oder Alkoxy mit 1 bis 6 Kohlenstoffatomen ist;

in Anwesenheit eines Katalysator, der einen Komplex aus Kupferchlorid, Rutheniumchlorid oder Rhodiumchlorid und Triphenylphosphin oder einen Komplex aus Kupferchlorid und Tri-n-butylphosphin umfaßt.

2. Verfahren nach Anspruch 1, worin die Reaktion bei einer Temperatur von 40° bis 200°C ausgeführt wird.

3. Verfahren nach Anspruch 2, worin die Temperatur zwischen 70° und 90°C liegt.

4. Verfahren nach Anspruch 1 bis 3, worin die Reaktion ohne Lösungsmittel durchgeführt wird.

5. Verfahren nach Anspruch 1, worin die Reaktanten in ungefähr äquimolaren Mengen verwendet werden.

**Revendications**

1. Procédé de préparation d'un composé de formule

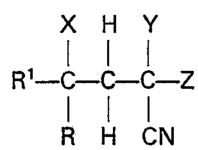

dans laquelle

R représente CHO ou $COR^2$ où $R^2$ est un halogène, alkyle comportant de 1 à 6 atomes de carbone ou alkoxy comportant de 1 à 6 atomes de carbone;

$R^1$ représente H ou un alkyle inférieur comportant de 1 à 6 atomes de carbone;

X représente Br, Cl ou I;

Y représente Cl, Br, $CF_3$, un alkyle comportant de 1 à 6 atomes de carbone, un aryle substitué ou non substitué, ayant de préférence de 6 à 10 atomes de carbone ou un radical hétérocyclique ayant 5 ou 6 atomes dans sons noyau; et

Z représente H, F ou Cl,

dans lequel on fait réagir un acétonitrile de formule

$$C(X^1)_3CN$$

dans laquelle

$X^1$ est n'importe quelles combinaison de H, Cl, Br, I, ou F, avec cette condition qu'il ne peut pas y avoir plus d'un H,

avec un composé oléfinique ayant la formule

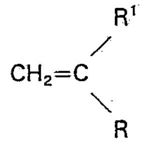

dans laquelle

$R^1$ représente H ou un alkyle inférieur comportant de 1 à 6 atomes de carbone; et

R représente CHO ou $COR^2$ où $R^2$ est un halogène, alkyle comportant de 1 à 6 atomes de carbone ou alkoxy comportant de 1 à 6 atomes de carbone;

en présence d'un catalyseur, qui comprend un complexe de chlorure cuivreux, chlorure de ruthénium ou chlorure de rhodium et de triphénylphosphine ou un complexe de chlorure cuivreux et de tri-n-butylphosphine.

2. Procédé de la revendication 1 dans lequel la réaction est effectuée à une température de 40° à 200°C.

3. Procédé de la revendication 2 dans lequel la température est 70° à 90°C.

4. Procédé des revendications 1 à 3 dans lequel la réaction est effectuée sans solvant.

5. Procédé de la revendication 1 dans lequel on emploie les réactifs en proportions molaires approximativement égales.